# EUROPEAN PATENT APPLICATION

(11) **EP 4 311 832 A1**
(43) Date of publication of application: **31.01.2024**
(21) Application number: 22186758.3
(22) Date of filing: 25.07.2022
(51) Int. Cl.: C07K 14/00

(54) **PHOTOCAGED OREXIN-B**

(71) Applicant: Universität Zürich, 8006 Zürich (CH)
(72) Inventor: HARTRAMPF, Nina, 8057 Zurich (CH); TATARSKIY, Petr, 8057 Zurich (CH); PATRIARCHI, Tommaso, 8057 Zurich (CH); DUFFET, Loïc, 8057 Zurich (CH)
(74) Representative: Schulz Junghans Patentanwälte PartGmbB

(57) **Abstract**

The invention relates to a photocaged peptide P-PC, with P being a peptide and PC a photocleavable segment, which is coupled to the peptide at its C-terminus and wherein the photocleavable segment PC is a compound of formula (I) 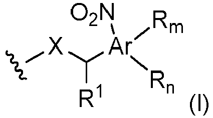 and method for synthesising the photocaged peptide P-PC.

## Description

### Field

The present invention relates to a photocaged peptide for control in signalling.

### Background

Orexins (orexin-A and orexin-B, also known as hypocretins) are neuropeptides produced selectively in neurons of the lateral hypothalamus and released throughout the central nervous system via long-range projections. In line with their widespread presence in brain areas and circuits, these peptides exert powerful control over brain function and physiology, as exemplified by the dramatic loss of stable wakefulness in orexin-deficient animals and humans. The pharmacological target of orexins are type-1 and type-2 orexin receptors (OX1R, OX2R, respectively). These receptors are members of the G-protein coupled receptor (GPCR) family and primarily couple to intracellular signaling via recruitment of G_{q}-proteins, with consequent activation of intracellular calcium activity that produces an excitatory effect on the orexin-receiving cells. The functional involvement of orexin signaling can be studied at the cellular, circuit, or whole-animal level using genetic knock-out models pharmacological agonists or antagonists of the receptors. However, such studies can only demonstrate the effect of slow and global activation or inhibition of orexin signaling, as tools to study spatiotemporally-controlled activation or inhibition of endogenous orexin receptors are not yet available.

Optogenetic and/or optochemical approaches are ideally suited for investigating neuropeptide signaling with high spatiotemporal precision. In this context, our recent work introducing a genetically-encoded fluorescent sensor for orexin neuropeptides allowed us for the first time to detect the spatial details of endogenous orexin signals as they spontaneously occur in intact animals, but lacked the ability to causally investigate orexin signaling. Synthetic light-responsive molecules, such as photoswitchable or photolabile groups, can overcome this limitation by using the high spatiotemporal precision of light for localized ligand activation. Photoswitchable derivatives reversibly change their configuration upon irradiation, and this change in conformation and polarity can impact target receptor binding affinity and efficacy. These photopharmacological tools have been widely exploited for the reversible optical control of small molecules (e.g. neurotransmitters), lipids and peptides/proteins, however, this approach requires permanent chemical modification of the native ligand. Photolabile groups (photocages), on the other hand, can be attached directly to the native ligand to sterically block ligand-receptor interactions, then irreversibly release (uncage) the native ligand upon light exposure. In peptide ligands, these photocages are typically attached to functional groups of amino acid side-chains, such as amines (Lys), carboxylic acids (Asp/Glu), amides (Asn/Gln), alcohols/phenols (Ser, Thr, Tyr) and thiols (Cys). Previous pioneering studies paved the way by introducing photocaged-neuropeptides to the optical toolbox (i.e. Leu-enkephalin, dynorphin-8). These studies demonstrated that precise control of neuropeptide release with light can be used as a powerful and complementary addition to the standard set of techniques used for investigating neuropeptide function on target cells and circuits.

Based on the above-mentioned state of the art, the objective of the present invention is to provide means and methods to photocaged peptides. This objective is attained by the subject-matter of the independent claims of the present specification, with further advantageous embodiments described in the dependent claims, examples, figures and general description of this specification.

### Summary of the Invention

A first aspect of the invention relates to a photocaged peptide P-PC, with P being a peptide and PC a photocleavable segment, which is coupled to the peptide at its C-terminus and wherein the photocleavable segment PC is a compound of formula (I) 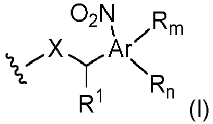 and wherein
X is NH or O,
Ar is an aromatic system selected from benzene, naphthalene, benzophenone, indole, benzothiophene, benzofuran, benzimidazole, quinoline, quinazoline, furan, thiophene, pyrrazole or pyrrole,
R¹ is a C₁-C₆-alkyl, -O-C₁-C₆-alkyl or a separated linker SL, particularly C₁-C₆-alkyl or the separated linker SL,
n is 0, 1, 2 or 3, particularly 1 or 2,
Rₘ is the separated linker SL and m is 0 or 1,
wherein at least one of R¹ or Rₘ is the separated linker SL,
Rₙ is selected from
   - -O-C₁₋₆, -N-C₁₋₃, -S-C₁₋₃,
   - the group of halogens,
   - C₅-C₆-aryl, C₅-C₆-heteroaryl,
   - C₂-C₉-conjugated alkenes, C₂-C₉-conjugated alkynes,
SL is -Y-SP-E, wherein Y is O, S, NR³ or CR³₂ and wherein R³ is H and/or a C₁-C₂ alkyl and wherein SP is a spacer comprising up to eight atoms, particularly carbon atoms, and wherein E is selected from -CONH₂, -COOH, -OH, -COSH, -COS-C₁-C₃-alkyl, - COS-C₅-C₆-aryl, -CONH-NH₂, -COO-C₁-C₄-alkyl, -COO-C₅-C₆-aryl, -CONH-C₁-C₂-alkyl, -CON-(C₁-C₂-alkyl)_{2.}

A second aspect of the invention relates to a method for synthesising the photocaged peptide P-PC particularly according to claims 1 to 13, wherein the method is characterised in that
a. an Fmoc protected amino acid building block (AA) is coupled to a double linker, wherein the double linker comprises a photocleavable segment precursor (PCP) of formula (VII), characterised in that it comprises a terminal moiety allowing the connection to a resin linker (RL) 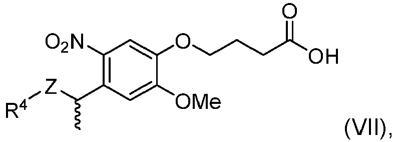 wherein Z is O or NH,
   R⁴ is Fmoc or H,
b. and the resin linker (RL), wherein in particular the resin linker is a a Rink amide linker, HMPB, Fmoc-PAL-OH or Fmoc-MeDBz-OH particularly Rink amide or HMPB, yielding a linked amino acid building block AA-PCP-RL,the linked amino acid building block is deprotected and coupled in continuous coupling and deprotection steps via automated solid-phase peptide synthesis, yielding a photocaged peptide precursor P-PCP-RL,
c. the photocaged peptide precursor is decoupled from the resin linker, yielding the photocaged peptide P-PC,

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *photocaged peptide* in the context of the present specification relates to a peptide according to the separate definition herein, which is attached to a molecular species that can be activated by light.

The term *photocleavable segment* in the context of the present specification relates to a chemical molecule or moiety attached to another substrate, for example a peptide, that can be removed from the substrate through light irradiation.

The term *separated linker SL* in the context of the present specification relates to the linker of the photocleavable segment which is obtained after cleavage from the resin linker.

The term *spacer SP* in the context of the present specification relates to a flexible chemical molecule or moiety that is used to link two molecules of interest together. A spacer can be of any length.

The term GPCR in the context of the present specification relates to G-protein coupled receptor.

The term *amino acid building block AA* in the context of the present specification relates to a single amino acid or short peptide comprising two to three amino acids, which are protected at their amino acid side chain(s) with any common protecting group used for amino acids. ^{R1}

The term *photocleavable peptide precursor PCP* in the context of the present specification relates to the photocleavable segment, which may or may not be protected and which is connected to the resin linker.

The term *resin linker RL* in the context of the present specification relates to the linker connecting the amino acid building block or peptide and photocleavable segment to the solid phase to allow the solid phase peptide synthesis to take place.

### General Biochemistry: Peptides, Amino Acid Sequences

The term *peptide* in the context of the present specification relates to a molecule consisting of up to 200 amino acids, in particular up to 100 amino acids, more particularly up to 50 amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

### Organic Chemistry

The term *C₅-C₆-aryl* in the context of the present specification relates to a cyclic aromatic hydrocarbon compound containing 5 to 6 carbon atoms. Examples include but are not limited to benzene or cyclopentadiene. A *C₅-C₆-aryl* in the context of the specification additionally may be substituted by one or more alkyl groups.

The term *C₅-C₆-heteroaryl* in the context of the present specification relates to a cyclic aromatic hydrocarbon compound that comprise one or more heteroatoms (e.g. N, O, S) and wherein the total number of atoms is 5 or 6. Examples include, without being restricted to, pyrrole, thiophene, furan, imidazole, pyrazole, thiazole, oxazole, pyridine, pyrimidine and thiazine.

The term *aryl* in the context of the present specification relates to a cyclic aromatic C₅-C₁₀ hydrocarbon that may comprise a heteroatom (e.g. N, O, S). Examples of aryl include, without being restricted to, phenyl and naphthyl, and any heteroaryl. A heteroaryl is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the specification additionally may be substituted by one or more alkyl groups.

The term *C₂-C₉ conjugated alkene* in the context of the present specification relates to a saturated linear hydrocarbon comprising one or more conjugated double bonds. An unsubstituted alkylene consists of C and H only. A substituted alkylene may comprise substituents as defined herein for substituted alkyl.

The term *C₂-C₉ conjugated alkyne* in the context of the present specification relates to a saturated linear hydrocarbon comprising one or more triple bonds and may also comprise one or more double bonds in addition to the triple bond(s) as long as the triple and/or double bonds are conjugated. An unsubstituted alkyne consists of C and H only. A substituted alkylyne may comprise substituents as defined herein for substituted alkyl.

The term *C₁-C₄ alkyl* in the context of the present specification relates to a saturated linear or branched hydrocarbon having 1, 2, 3 or 4 carbon atoms, wherein in certain embodiments one carbon-carbon bond may be unsaturated and one CH₂ moiety may be exchanged for oxygen (ether bridge) or nitrogen (NH, or NR with R being methyl, ethyl, or propyl; amino bridge). Non-limiting examples for a *C₁-C₄* alkyl are methyl, ethyl, propyl, prop-2-enyl, n-butyl, 2-methylpropyl, *tert*-butyl, but-3-enyl, prop-2-inyl and but-3-inyl. In certain embodiments, a C₁-C₄ alkyl is a methyl, ethyl, propyl or butyl moiety.

A *C₁-C₆ alkyl* in the context of the present specification relates to a saturated linear or branched hydrocarbon having 1, 2, 3, 4, 5 or 6 carbon atoms. Non-limiting examples for a *C₁-C₆* alkyl include the examples given for *C₁-C₄* alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, pent-4-inyl, 3-methyl-2-pentyl, and 4-methyl-2-pentyl. In certain embodiments, a C₅ alkyl is a pentyl or cyclopentyl moiety and a C₆ alkyl is a hexyl or cyclohexyl moiety.

The term *substituted alkyl* in its broadest sense refers to an alkyl as defined above in the broadest sense, which is covalently linked to an atom that is not carbon or hydrogen, particularly to an atom selected from N, O, F, B, Si, P, S, Cl, Br and I, which itself may be -if applicable- linked to one or several other atoms of this group, or to hydrogen, or to an unsaturated or saturated hydrocarbon (alkyl or aryl in their broadest sense). In a narrower sense, *substituted alkyl* refers to an alkyl as defined above in the broadest sense that is substituted in one or several carbon atoms by groups selected from amine NH₂, alkylamine NHR, imide NH, alkylimide NR, amino(carboxyalkyl) NHCOR or NRCOR, hydroxyl OH, oxyalkyl OR, oxy(carboxyalkyl) OCOR, carbonyl O and its ketal or acetal (OR)₂, nitril CN, isonitril NC, cyanate CNO, isocyanate NCO, thiocyanate CNS, isothiocyanate NCS, fluoride F, choride Cl, bromide Br, iodide I, phosphonate PO₃H₂, PO₃R₂, phosphate OPO₃H₂ and OPO₃R₂, sulfhydryl SH, suflalkyl SR, sulfoxide SOR, sulfonyl SO₂R, sulfanylamide SO₂NHR, sulfate SO₃H and sulfate ester SO₃R, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified.

### Detailed Description of the Invention

A first aspect of the invention relates to a photocaged peptide P-PC, with P being a peptide and PC a photocleavable segment, which is coupled to the peptide at its C-terminus and wherein the photocleavable segment PC is a compound of formula (I) 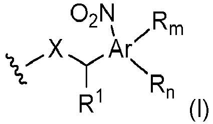 and wherein
X is NH or O,
Ar is an aromatic system selected from benzene, naphthalene, benzophenone, indole, benzothiophene, benzofuran, benzimidazole, quinoline, quinazoline, furan, thiophene, pyrrazole or pyrrole,
R¹ is a C₁-C₆-alkyl, -O-C₁-C₆-alkyl or a separated linker SL, particularly C₁-C₆-alkyl or the separated linker SL,
n is 0, 1, 2 or 3, particularly 1 or 2,
Rₘ is the separated linker SL and m is 0 or 1,
wherein at least one of R¹ or Rₘ is the separated linker SL,
Rₙ is selected from
   - -O-C₁₋₆, -N-C₁₋₃, -S-C₁₋₃,
   - the group of halogens,
   - C₅-C₆-aryl, C₅-C₆-heteroaryl,
   - C₂-C₉-conjugated alkenes, C₂-C₉-conjugated alkynes,
SL is -Y-SP-E, wherein Y is O, S, NR³ or CR³₂ and wherein R³ is H and/or a C₁-C₂ alkyl and wherein SP is a spacer comprising up to eight atoms, particularly carbon atoms, and wherein E is selected from -CONH₂, -COOH, -OH, -COSH, -COS-C₁-C₃-alkyl, - COS-C₅-C₆-aryl, -CONH-NH₂, -COO-C₁-C₄-alkyl, -COO-C₅-C₆-aryl, -CONH-C₁-C₂-alkyl, -CON-(C₁-C₂-alkyl)_{2.}

Photolabile groups (photocages), can be attached directly to a native ligand to sterically or electronically block ligand-receptor interactions, then irreversibly release (uncage) the native ligand upon light exposure. In peptide ligands, these photocages are typically attached to functional groups of amino acid side-chains, such as amines (Lys), carboxylic acids (Asp/Glu), amides (Asn/Gln), alcohols/phenols (Ser, Thr, Tyr) and thiols (Cys).

Increasing the red shift by using appropriate substituents at the Rₙ position or by using appropriate moieties, such as conjugated double bonds within the separated linker results in the use of a less toxic irradiation wavelength and by that makes the photocage a more versatile molecule for the use in cellular based assays. Additionally spectral multiplexing with other light-sensitive tools can be enabled.

Using -O-C₁₋₆-N-C₁₋₃, or -S-C₁₋₃ as residue at the Rₙ position increases the red shift through their electron pushing effect.

Using halogens as residue at the Rₙ position results in higher stability of the aromatic system through the electron withdrawing effect of halogens and the resulting loss in reactivity. Additionally, they may increase the mesomeric effect by enlarging the space for the π-electrons and by that increase the red shift.

Using -C₅-C₆-aryl, -C₅-C₆-heteroaryl, -C₂-C₉-conjugated alkenes or -C₂-C₉-conjugated alkynes as substituents increases the red shift of the compound through the extension of the aromatic system by further aromatic substituents or further substituents of linear conjugated double bonds.

In certain embodiments the photocleavable segment PC is a compound of formula (II) 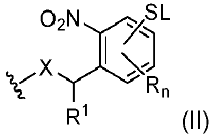 and wherein
X is NH or O,
R¹ is C₁-C₆-alkyl,
n is 0, 1, 2 or 3, particularly 1 or 2, particularly 1,
Rₙ is selected from
   - -O-C₁₋₆, -N-C₁₋₃, -S-C₁₋₃,
   - the group of halogens,
   - C₅-C₆-aryl, C₅-C₆-heteroaryl,
   - C₂-C₉-conjugated alkenes, C₂-C₉-conjugated alkynes,
SL is -Y-SP-E, wherein Y is O, S, NR³ or CR³₂ and wherein R³ is H and/or a C₁-C₂ alkyl and wherein SP is a spacer comprising up to eight atoms, particularly carbon atoms, and wherein, and wherein E is selected from -CONH₂, -COOH, -OH, -COSH, -COS-C₁-C₃-alkyl, -COS-C₅-C₆-aryl, -CONH-NH₂, -COO-C₁-C₄-alkyl, -COO-C₅-C₆-aryl, -CONH-C₁-C₂-alkyl, -CON-(C₁-C₂-alkyl)_{2.}

A longer spacer SP can comprise several conjugated double bonds which extend the aromatic system of the benzene and by that increase the compounds red shift. An increased red shift results in the use of a less toxic irradiation wavelength and by that makes the photocage a more versatile molecule for the use in cellular based assays. Additionally spectral multiplexing with other light-sensitive tools can be enabled.

Using -O-C₁₋₆-N-C₁₋₃, or -S-C₁₋₃ as residue at the Rₙ position increases the redshift through their electron pushing effect.

Using halogens as residue at the Rₙ position results in higher stability of the aromatic system through the electron withdrawing effect of halogens and the resulting loss in reactivity. Additionally, they may increase the mesomeric effect by enlarging the space for the π-electrons and by that increase the red shift.

Using -C₅-C₆-aryl, -C₅-C₆-heteroaryl, -C₂-C₉-conjugated alkenes or -C₂-C₉-conjugated alkynes as substituents increases the red shift of the compound through the extension of the aromatic system by further aromatic substituents or further substituents of linear conjugated double bonds.

In certain embodiments, R¹ is selected from C₁-C₆ alkyl.

In certain embodiments, R¹ is selected from C₁-C₃ alkyl.

In certain embodiments, R¹ is methyl or ethyl.

In certain embodiments, R¹ is methyl.

In certain embodiments, Y is O, NR³ or CR³₂,

In certain embodiments, Y is O or CR³₂.

In certain embodiments, Y is O.

In certain embodiments, SP comprises 2 to 6 atoms, particularly carbon atoms.

By using a spacer with carbon atoms, the spacer may comprise no double bonds, or 1 to 3 double bonds to increase the compounds red shift.

In certain embodiments, SP comprises 2 to 4 atoms, particularly carbon atoms.

In certain embodiments, SP comprises 3 atoms, particularly carbon atoms.

In certain embodiments, E is selected from -CONH₂, -COOH, -OH, -COO-C₁-C₄-alkyl, or - COO-C₅-C₆-aryl, -COS-C₁-C₄-alkyl, or -COS-C₅-C₆-aryl, particularly from -CONH₂, -COOH, - OH or -COO-C₁-C₄-alkyl, more particularly E is -CONH₂ or -COOH.

In certain embodiments, Rₙ is selected from
- -O-C₁₋₆, -N-C₁₋₃,
- the group of halogens,
- C₂-C₉-conjugated alkenes, C₂-C₉-conjugated alkynes.

In certain embodiments, Rₙ is selected from
- -O-C₁₋₆, -N-C₁₋₃,
- the group of halogens,
- C₂-C₉-conjugated alkenes.

In certain embodiments, Rₙ is selected from
- -O-C₁₋₆, -N-C₁₋₃,
- C₂-C₉-conjugated alkenes.

In certain embodiments, Rₙ is -O-C₁₋₆, or -N-C₁₋₃.

In certain embodiments, Rₙ is -O-C₁₋₃ or -N-C₁₋₂.

In certain embodiments, Rₙ is -O-C₁₋₃.

In certain embodiments, Rₙ is -O-C₁₋₂.

In certain embodiments, Rₙ is OMe.

In certain embodiments, the photocleavable segment is a compound of formula (III), (IV), (V) or (VI)

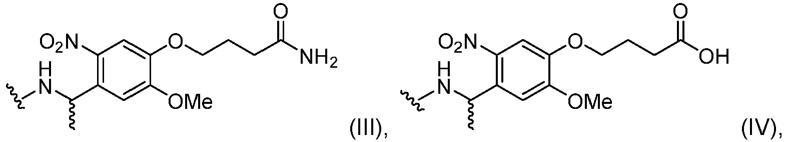

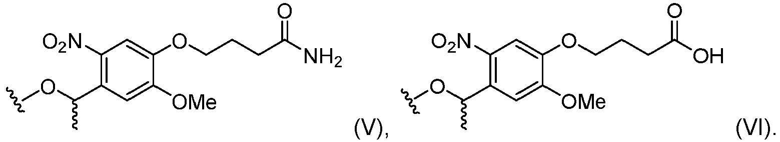

In certain embodiments, the photocleavable segment is a compound of formula (III) or (V).

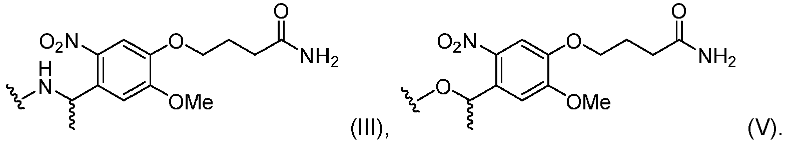

In certain embodiments, the photocleavable segment is a compound of formula (III)

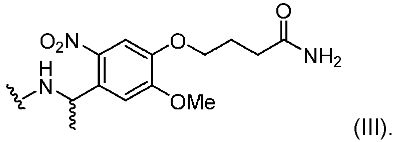

The photocaged peptide P-PC can activate endogenous peptide receptors in response to 405 nm illumination and is compatible with implantable wireless optofluidic devices for in vivo optical control of endogenous peptide receptors with fine spatiotemporal precision.

In certain embodiments, the peptide P comprises a C-terminal amide or C-terminal carboxylic acid.

C-terminal amides or carboxylic acids on peptides are essential for their binding, thus, modifying the C-terminus of a peptide using a photocage will have an impact on its binding ability and present a useful tool for inhibition studies.

In certain embodiments, the peptide P is a receptor binding peptide.

In certain embodiments, the peptide P is a GPCR binding peptide.

In certain embodiments, P is selected from cholecycystokinin, gastrin, prolactin-releasing peptide, pyroglutamylated RF amide peptide, neuropeptide FF, kisspeptin, vasopressin, oxytocin, bombesin, gastrin-releasing peptide, neuromedin U, substance P, neurokinin, thyrotropin-releasing hormone, orexin A, apelin, endotelin, compliment ligand C3a, compliment ligand C5a, gonadoptrin releasing hormone, PDZ ligands or orexin B.

In certain embodiments, the peptide P is selected from neuropeptide Y, vasopressin, oxytocin, orexin A or orexin B.

Orexins (orexin-A and orexin-B, also known as hypocretins) are neuropeptides produced selectively in neurons of the lateral hypothalamus and released throughout the central nervous system via long-range projections. In line with their widespread presence in brain areas and circuits, these peptides exert powerful control over brain function and physiology, as exemplified by the dramatic loss of stable wakefulness in orexin-deficient animals and humans. The pharmacological target of orexins are type-1 and type-2 orexin receptors (OX1R, OX2R, respectively). These receptors are members of the G-protein coupled receptor (GPCR) family and primarily couple to intracellular signaling via recruitment of Gq-proteins, with consequent activation of intracellular calcium activity that produces an excitatory effect on the orexin-receiving cells. The functional involvement of orexin signaling can be studied at the cellular, circuit, or whole-animal level using genetic knock-out models, pharmacological agonists or antagonists, of the receptors.

In certain embodiments, the peptide P is orexin B.

Orexin-B (OXB) is a 28-amino acid neuropeptide that binds the orexin receptors (OXR) via the OXB C-terminus. Modification of amino acids in this region has a large impact on the ability of the peptide to trigger OXR activation, presenting an opportunity for the installation of a photocage.

A second aspect of the invention relates to a method for synthesising the photocaged peptide P-PC particularly according to claims 1 to 13, wherein the method is characterised in that
d. an Fmoc protected amino acid building block (AA) is coupled to a double linker, wherein the double linker comprises a photocleavable segment precursor (PCP) of formula (VII), characterised in that it comprises a terminal moiety allowing the connection to a resin linker (RL) 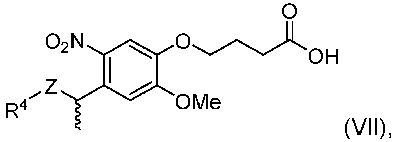 wherein Z is O or NH,
   R⁴ is Fmoc or H,
e. and the resin linker (RL), wherein in particular the resin linker is a Rink amide linker, HMPB, Fmoc-PAL-OH or Fmoc-MeDBz-OH particularly Rink amide or HMPB, yielding a linked amino acid building block AA-PCP-RL, the linked amino acid building block is deprotected and coupled in continuous coupling and deprotection steps via automated solid-phase peptide synthesis, yielding a photocaged peptide precursor P-PCP-RL,
f. the photocaged peptide precursor is decoupled from the resin linker and side-chain protecting groups are removed, yielding the photocaged peptide P-PC,
wherein all definitions are identical to the definitions of claims 1 to 13.

The method describes a "double linker" strategy for solid phase peptide synthesis, wherein the Photo linker would serve as the photocage, and a Rink amide linker would release the final photocaged peptide from the resin under acidic cleavage conditions.

In certain embodiments, Z is NH.

In certain embodiments, R⁴ is Fmoc.

In certain embodiments, n amino acid building blocks were used in the continuous coupling step, wherein n is 1 to 30.

In certain embodiments, n is 3 to 20.

In certain embodiments, n is 3 to 10.

In certain embodiments, the double linker is coupled to the C-terminus of the amino acid building block.

The invention is further illustrated by the following examples and figures, from which further embodiments and advantages can be drawn. These examples are meant to illustrate the invention but not to limit its scope.

### Description of the Figures

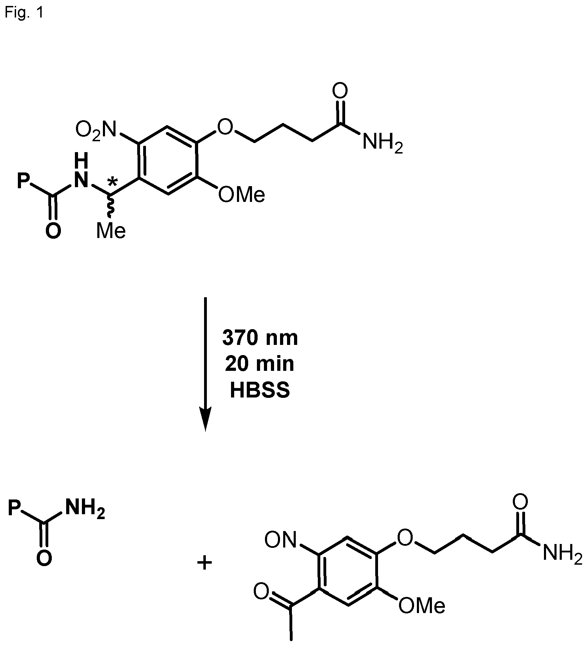

- Fig. 1: shows a schematic of photolysis reaction producing WT OXB from photo-OXB (calculated monoisotopic masses in brackets) and the corresponding photocage; * denotes the chiral center on the photocaging group; P is the peptide according to the definitions throughout the application.

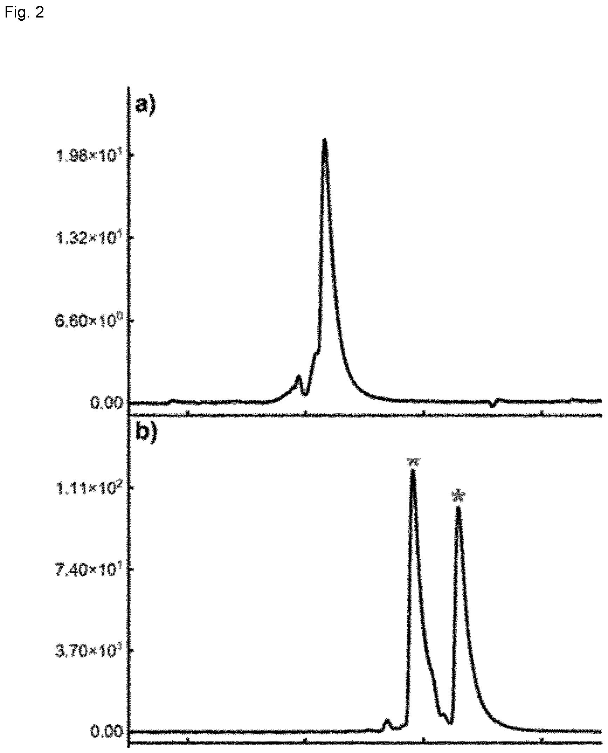

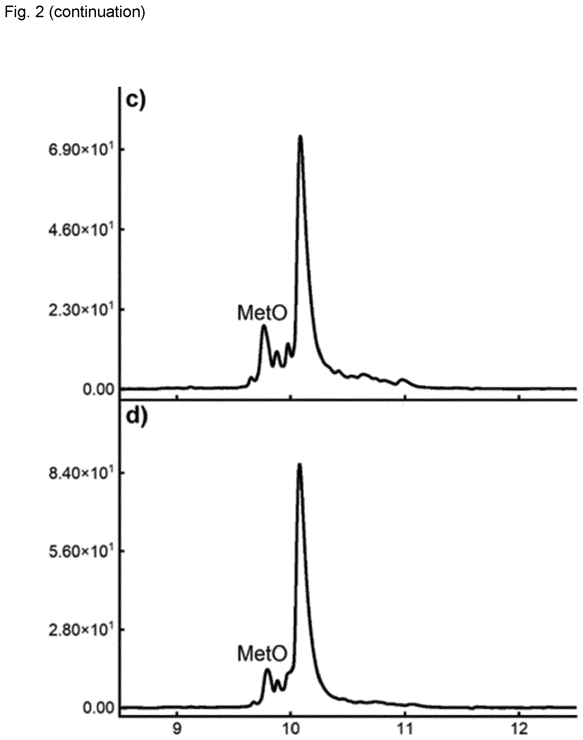

- Fig. 2: shows the photolysis (uncaging) of photo-OXB releases WT OXB. UHPLC traces at 214 nm of a) WT OXB, b) Photo-OXB before photolysis; the two signals correspond to two diastereomers arising from the chiral center of the photocaging group (denoted with *), c) Photolyzed photo-OXB (370 nm, 20 min, room temperature), d) Co-injection of photolyzed photo-OXB and WT OXB; MetO denotes oxidation of M28 to methionine sulfoxide; Y-axis: Response [mAu], X-axis: time [min].

### Examples

### Example 1:

### All-optical assay for the in vitro characterisation of orexin-B uncaging

To characterize the sensitivity of the photo-OXB molecule to 405 nm laser light the inventors established an all-optical assay in which OxLight1-expressing cells were bathed in a solution containing photo-OXB (1 µM) and sensor fluorescence was measured before and after uncaging (405 nm laser, 0.38 mW). To assess the uncaging efficiency of the photo-OXB, titration of light pulses in increasing duration with constant laser power and uncaging area was performed. Under these conditions a graded response of OxLight1, in which the maximal fluorescence response (ΔF/F₀) scaled with the duration of uncaging was observed, indicating that the release of OXB peptide could be fine-tuned by changing the duration of uncaging with UV light. In contrast, no sensor response was observed in a control experiment, in which the OxLight1-expressing cells were treated with the illumination protocol in the absence of photo-OXB. These results demonstrate that biosensor imaging using OxLight1 during photo-OXB uncaging can be used as a sensitive and specific in vitro all-optical assay to determine the efficacy of photo-OXB uncaging.

During these experiments, a spatial directionality of the biosensor response to photo-OXB uncaging which gradually spread outwards from the center of the illuminated, uncaging area in circular wave-like pattern was observed. To further investigate this, the spatial profile of OxLight1 activation upon photo-OXB uncaging was quantified. OxLight1 the ΔF/F response upon uncaging (405 nm laser, 0.38 mW, 20 sec) in circular bands centered in the uncaging area and emanating outwards in 10 µm-wide steps was recorded. It was observed that the photoreleased OXB diffused slowly (the shortest *τ* measured in our assay was ~5 sec) and gradually across the imaged area, as reflected by the increased activation time constant of OxLight1 measured at increasing distance from the uncaging area. The results clearly show that in vitro photo-OXB uncaging triggers a circular wave-like propagation pattern of photoreleased OXB originating from the center of uncaging and emanating over the surrounding cells, and that this phenomenon can be detected by imaging OxLight1-expressing cells.

### Example 2:

### Functional effect of orexin-B uncaging in vitro

Orexin receptors signal through G_{q}-proteins and subsequent intracellular calcium release¹⁰. To assess whether the photoreleased OXB was able to exert its biological activity after uncaging, we recorded intracellular calcium signals in HEK293T cells co-expressing the wild-type OXR2 and the red genetically-encoded calcium sensor jRGECO1a. We first recorded baseline cellular calcium activity, then bath-applied photo-OXB (1 nM, based on our previous experience with this cell-based assay) to the cells during imaging and, following a brief delay period, used UV light to illuminate a small area at the center of the field of view (405 nm laser, 0.38 mW, 10 sec) to trigger OXB release onto the cells. Addition of photo-OXB without uncaging did not trigger any noticeable increase in cellular calcium activity. In contrast, a strong increase in calcium activity was observed shortly after illumination - and thereby release of OXB - began. The brief delay between illumination (uncaging of photo-OXB) and the increase in calcium activity is presumably due to the time required for diffusion of the released OXB and for signaling to occur. Importantly, cellular calcium activity was almost completely absent upon photo-OXB uncaging on HEK293T cells that did not express the OXR2. This indicated that the OXB-mediated increase in intracellular calcium activity, as controlled by photo-OXB uncaging, resulted from the specific activation of OX2R. These results demonstrate that our photo-OXB construct can be used to release biologically active OXB upon uncaging with 405 nm light and trigger orexin receptor-mediated intracellular signaling in vitro.

### Example 3:

### Functional effect of orexin-B uncaging in acute brain slices

The perfusion of orexin, or the optogenetic activation of orexinergic projections in acute brain slices have been shown to trigger a membrane depolarization effect in type-2 dopamine receptor (D2R)-expressing medium spiny neurons (D2-MSNs) of the Nucleus Accumbens (NAc), without affecting the membrane potential of type-1 dopamine receptor (D1R)-expressing MSNs (D1-MSNs). We thus set out to evaluate the potential utility of our newly developed photo-OXB construct in ex vivo experiments, using depolarization of the membrane potential as a readout. To do so, we prepared acute brain slices containing NAc from wild-type mice and recorded membrane potentials in MSNs. It has been demonstrated that in the NAc and the dorsal striatum D1-MSNs have lower input resistance and firing rate than D2-MSNs. We used this electrophysiological signature to identify D1 and D2-MSNs. After the cell type was identified, the effect of uncaging of photo-OXB was evaluated by full-field illumination of the slice using a 385 nm LED light (2 mW/mm²). In the absence of photo-OXB, UV illumination triggered a small dip of the membrane potential in both D1 and D2-MSNs, but the membrane potential quickly recovered back to baseline levels. The membrane potential of D1-MSNs displayed a similar response to the UV illumination when the artificial cerebrospinal fluid (aCSF) contained photo-OXB (300 nM). However, UV illumination evoked a clear depolarization of the membrane potential in D2-MSNs in the presence of photo-OXB in the aCSF. The cell type-specific depolarization we obtained by the uncaging of photo-OXB is consistent with the results previously observed with the bath application of orexin or optogenetic activation of orexin terminals, confirming that photo-OXB can be effectively utilized to trigger endogenous orexin receptor activation and subsequent membrane depolarization effects in D2R-MSNs with high temporal precision.

### Example 4:

### Sequences

**Table 1: shows sequences used herein. * relates to a photo caging (PC) modification.**

| Name | Sequence | SEQ ID NO: |
|---|---|---|
| OXB (orexin B) | RSGPPGLQGRLQRLLQASGNHAAGILTM | 1 |
| Photo-OXB (photo-orexin B) | RSGPPGLQGRLQRLLQASGNHAAGILTM* | 2 |
| OX1R (orexin receptor type 1) | MEPSATPGAQMGVPPGSREPSPVPPDYEDEFLRYLWRD YLYPKQYEWVLIAAYVAVFVVALVGNTLVCLAVWRNHHM RTVTNYFIVNLSLADVLVTAICLPASLLVDITESWLFGHALC KVIPYLQAVSVSVAVLTLSFIALDRWYAICHPLLFKSTARRA RGSILGIWAVSLAIMVPQAAVMECSSVLPELANRTRLFSV CDERWADDLYPKIYHSCFFIVTYLAPLGLMAMAYFQIFRKL WGRQIPGTTSALVRNWKRPSDQLGDLEQGLSGEPQPRA RAFLAEVKQMRARRKTAKMLMVVLLVFALCYLPISVLNVL KRVFGMFRQASDREAVYACFTFSHWLVYANSAANPIIYNF LSGKFREQFKAAFSCCLPGLGPCGSLKAPSPRSSASHKS LSLQSRCSISKISEHVVLTSVTTVLP | 3 |
| OX2R (orexin receptor type 2) | MSGTKLEDSPPCRNWSSASELNETQEPFLNPTDYDDEEF LRYLWREYLHPKEYEWVLIAGYIIVFVVALIGNVLVCVAVW KNHHMRTVTNYFIVNLSLADVLVTITCLPATLVVDITETWFF GQSLCKVIPYLQTVSVSVSVLTLSCIALDRWYAICHPLMFK STAKRARNSIVIIWIVSCIIMIPQAIVMECSTVFPGLANKTTL FTVCDERWGGEIYPKMYHICFFLVTYMAPLCLMVLAYLQI FRKLWCRQIPGTSSVVQRKWKPLQPVSQPRGPGQPTKS RMSAVAAEIKQIRARRKTARMLMIVLLVFAICYLPISILNVLK RVFGMFAHTEDRETVYAWFTFSHWLVYANSAANPIIYNFL | 4 |
| | SGKFREEFKAAFSCCCLGVHHRQEDRLTRGRTSTESRKS LTTQISNFDNISKLSEQVVLTSISTLPAANGAGPLQNW | |

### Material and methods

### Chemicals and solvents

All chemicals and solvents were used as supplied, unless otherwise stated. Fmoc- and side chain-protected L-amino acids (Fmoc-Ala-OH, Fmoc-Arg(Pbf)-OH, Fmoc-Asn(Trt)-OH, Fmoc-Asp(O*t*-Bu)-OH, Fmoc-Cys(Trt)-OH, Fmoc-Gln(Trt)-OH, Fmoc-Glu(Ot-Bu)-OH, Fmoc-Gly-OH, Fmoc-His(Trt)-OH, Fmoc-Ile-OH, Fmoc-Leu-OH, Fmoc-Lys(Boc)-OH, Fmoc-Met-OH, Fmoc-Phe-OH, Fmoc-Pro-OH, Fmoc-Ser(*t*-Bu)-OH, Fmoc-Thr(*t*Bu)-OH, Fmoc-Trp(Boc)-OH, Fmoc-Tyr(*t*-Bu)-OH, Fmoc-Val-OH) were purchased from Bachem AG or the Novabiochem-line from Sigma-Aldrich Chemie GmbH; (Pbf = 2,2,4,6,7-pentamethyldlhydrobenzofuran-5-sulfonyl, Trt = trityl, *t*-Bu = *tert*-butyl, Boc = *tert*-butoxycarbonyl). Boc-Lys-OH, 1-(2-nitrophenyl)ethanol, Fmoc N-Hydroxysuccinimidester (Fmoc-OSu), and piperidine were purchased from Chemie Braunschwig AG. *O*-(7-Azabenzotriazol-1-yl)-*N,N,N',N*'-tetramethyluronium hexafluorophosphate (HATU) and (7-azabenzotriazol-1-yloxy)tripyrrolidinophosphonium hexafluorophosphate (PyAOP) were purchased from Bachem AG and Advanced ChemTech CreoSalus^{®}, respectively; 4-{4-[1-(9-fluorenylmethyloxycarbonylamino)ethyl]-2-methoxy-5-nitrophenoxy}butanoic acid (Fmoc-Photo linker) was purchased from Iris Biotech GmbH; N,N-diisopropylethylamine (*i*-Pr₂NEt, DIPEA, 99.5%), trifluoroacetic acid (TFA, for HPLC, ≥99.0%), triisopropylsilane (TIPS, 98%) and 3,6-dioxa-1,8-octane-dithiol (DODT, 95%), bis(trichlormethyl)carbonate, were purchased from Sigma-Aldrich Chemie GmbH; N,N-dimethylformamide (DMF) was purchased from the Supelco-line from VWR International GmbH; dichloromethane (DCM, ≥99.8%) was purchased from Fischer Scientific Inc.; diethyl ether was purchased from Honeywell Riedel-de Haën; acetonitrile (MeCN, HPLC gradient grade, ≥99.9%) was purchased from Sigma-Aldrich Chemie GmbH. Dry tetrahydrofuran (THF, without stabilizer) was purchased from Sigma-Aldrich Chemie GmbH and dried in a Pure Solvent System, dry 1,4-dioxane was purchased from Chemie Braunschwig AG. Solvents for flash column chromatography and crystallization experiments were purchased in technical grade and distilled under reduced pressure prior to use. NovaPEG Rink Amide resin (0.41 mmol/g loading, Lot: S7863347 038) was purchased from the Novabiochem-line and AldraAmine trapping packets (volume 1000-4000 mL) were purchased from Sigma-Aldrich Chemie GmbH.

### NMR Spectroscopy

Nuclear magnetic resonance spectra were recorded in deuterated chloroform (CDCl₃) or D₂O in 5 mm tubes on a Bruker AV2-401 (400 MHz) spectrometer equipped with a BOSS-I shim system, a digital lock control unit, an AMOS Control System, a DQD unit, a BVT3200 with a BCU05 cooling unit, GRASP Level II for gradient spectroscopy. Chemical shifts (δ scale) are expressed in parts per million (ppm) and are calibrated using residual protic solvent as an internal reference (CHCl₃: *δ* = 7.26 ppm, H₂O: *δ* = 4.79 ppm. Data for ¹H NMR spectra are reported as follows: chemical shift (*δ* ppm) (multiplicity, coupling constants (Hz), integration). Couplings are expressed as: s = singlet, d = doublet, t = triplet, q = quartet, m = multiplet or combinations thereof. Carbon nuclear magnetic resonance (¹³C NMR) spectra were recorded at 75, 100 and 150 MHz, respectively. Carbon chemical shifts (*δ* scale) are also expressed in parts per million (ppm) and are referenced to the central carbon resonances of the solvents (CHCl₃: δ = 77.2 ppm).

### UV-Vis Spectroscopy

UV-Vis spectra were acquired using a Multiskan SkyHigh Microplate Spectrophotometer (ThermoFisher Scientific Inc.) in the range of 200-900 nm with 1 nm steps and quartz cuvettes from Hellma Schweiz AG (10 mm pathlength).

### AFPS Peptide Synthesis

Peptides were synthesized on an automated-flow system, which was built in the Hartrampf lab, based on the published AFPS system. All DMF used was treated with AldraAmine trapping packets for at least 24 hours before experiments. Capitalized letters refer to L-amino acids. Unless otherwise noted, the following settings were used for peptide synthesis: flow rate = 20 mL/min for coupling and deprotection steps, 40 mL/min for washing, temperature = 90 °C (loop) and 85-90 °C (reactor). The used system is differs in the following settings form the previously reported: All three pumps are then actuated together for a period of three pumping strokes (for amino acids D, E, F, G, I, K, L, M, P, S, W, Y) or eight pumping strokes (for amino acids A, C, H, N, Q, R, S, T, V) for the coupling step.

### Cleavage Protocol

After synthesis, the peptidyl resin was washed with DCM (3 × 5 mL), dried under reduced pressure, and weighed. The resin (amount as specified) was then transferred into a 3 mL polypropylene (PP) syringe equipped with a 20 µm pore size frit. Approximately 1.5 mL of cleavage solution (94% TFA, 1% TIPS, 2.5% EDT, 2.5% H₂O, *v*/*v*/*v*/*v)* was added to the syringe. If needed, more cleavage solution was added to ensure complete submersion of the resin. The capped syringe was shaken at room temperature for 2 h. Subsequently, the solution was filtered into a 15 mL conical PP tube, followed by evaporation of approximately 2/3 of the cleavage solution by a light stream of N₂. Thereafter, ice-cold diethyl ether (14 mL) was added to the cleavage mixture and the precipitate was collected by centrifugation and triturated twice more with ice-cold diethyl ether (14 mL). The supernatant was discarded. Residual ether was allowed to evaporate, and the peptide was dissolved in 50% acetonitrile in water with 0.1% TFA. The peptide solution was frozen, lyophilized until dry, and weighed.

### Analytical LC-HR-ESI-MS

Liquid chromatography high resolution electrospray ionization mass spectrometry (LC-HR-ESI-MS): Acquity UPLC (Waters, Milford, USA) connected to an Acquity eλ diode array detector and a Synapt G2HR-ESI-QTOF-MS (Waters, Milford, USA); injection of 10 µL sample (c= ca. 10-100 µg/mL in the indicated solvent); Acquity BEH C8 HPLC column (1.7 µm particle size, 2.1 × 100 mm, Waters) kept at room temperature; elution at a flow rate of 0.3 mL/min with A: H₂O + 0.02% HCO₂H + 0.04% CF₃CO₂H and B: CH₃CN + 0.04% HCO₂H + 0.02% CF₃CO₂H, isocratic 5% B for 1 min; then 5-95% B over 9 min. UV-Vis spectra recorded in the range of 190-300 nm at 1.2 nm resolution and 20 points s⁻¹; ESI: positive ionization mode, capillary voltage 3.0 kV, sampling cone 40 V, extraction cone 4 V, N₂ cone gas 4 L/h, N₂ desolvation gas 800 L/min, source temperature 120 °C; mass analyzer in resolution mode: mass range 150-3000 *m*/*z* with a scan rate of 1 Hz; mass calibration to < 2 ppm within 50-2500 *m*/*z with* a 5 mM aq. soln. of HCO₂Na, lock masses: *m*/*z* 195.0882 (caffein, 0.7 ng/mL) and 556.2771 (Leucine-enkephalin, 2 ng/mL).

All mass spectra are integrated across R*ₜ* = 4-6 min of the total ion count (TIC). The areas indicated in grey are excluded from integration, due to presence of the injection peak and solvent-mixing baseline perturbation. For purity of the final peptides, please refer to the UHPLC spectra. Deconvoluted masses from the raw *m*/*z* values are calculated using Mestrelab Research S.L.^{©}MestReNova v. 14.1 Mnova MS Suite.

### Analytical Ultra High Performance Liquid Chromatography (UHPLC)

For determination of purity by UHPLC, the filtered peptide solution was diluted in 10-50% acetonitrile (MeCN) in water with 0.1% TFA (500 µL) to a final concentration of approximately 0.5 mg/mL. The samples were analyzed on Agilent 1290 Infinity II Series, which was computer-controlled through Agilent OpenLab CDS and ChemStation software.

For standard analysis of all peptide samples, analytical UHPLC spectra were recorded on an analytical Agilent Zorbax 300SB-C18 Rapid Resolution HD column (2.1 mm × 100 mm, 1.8 µm particle size) kept at 40°C; elution at a flow rate of 0.8 mL/min with A: MeCN/H₂O (5:95) + 0.1% TFA and B: MeCN/H₂O (95:5) + 0.1% TFA, isocratic 0% B for 3 min; then 0-100% B over 20 min (ca. 4.5% MeCN/min) with UV detection at 214 nm and 120 points s⁻¹. The total method time was 23.1 min. Then, the column was re-equilibrated using a post-run method at 0% Solvent B for 2 min. Purities of the purified peptides were calculated by integration of the Area Under the Curve (AUC) of desired product peak as a percentage of the AUC of all peaks (within 3-18 min) at A = 214 nm (amide backbone).

### Semi-preparative Reverse-Phase High Performance Liquid Chromatography (RP-HPLC)

Semi-preparative RP-HPLC was performed on a Shimadzu prominence HPLC system (Shimadzu Corp., Japan) with a CBM-40 system controller module, an FRC-10A fraction collector, two LC-20AR pumps, and an SPD-40 UV/VIS detector, using an Agilent Zorbax 300SB-C18 Semi-Preparative column (9.4 × 250 mm, 5 µm particle size) kept at room temperature; elution at a flow rate of 4 mL/min with A: H₂O + 0.1% TFA, and B: MeCN + 0.1% TFA. Purifications were executed using the following method: isocratic 5% B for 5 min, then 5-30% B over 25 min (ca. 1 %B/min), 30-38% B over 40 min (ca. 0.2%B/min), then 38-65% B over 14 min (ca. 2%B/min), 65-95% over 1 min, and isocratic 95% for 14 min.

### Synthesis of the double linker (Fmoc-Photolinker + Resin linker)

To a suspension of Fmoc-Photo linker (51 mg, 98 µmol, 3.0 eq.) and PyAOP (0.38 M, 0.25 mL, 98 µmol, 3.0 eq.) in DMF (0.73 mL, 0.10 M final concentration) was added DIPEA (0.74 mL, 0.20 mmol, 6.0 eq.), resulting in an immediate dissolution of all components. Subsequently, this mixture was added to a syringe charged with ChemMatrix Rink amide resin (80 mg, 32 µmol, 1.0 eq.) and allowed to react under gentle agitation for 3 h at room temperature in the dark (note: from here on the peptidyl resin and the resulting products were protected from light). Thereafter, (I) the resin was washed with DMF (3 × 5 mL) and the peptide was synthesized immediately, or (II) the resin was washed with DMF (3 × 5 mL), DCM (3 × 5 mL), dried under reduced pressure and stored in the dark.

### Synthesis of Photo-OXB

The resin with the double linker was swollen with DMF (3 × 5 mL) followed by peptide synthesis following standard conditions described in AFPS Peptide Synthesis with the following changes: synthesis commenced with Fmoc deprotection at 60 mL/min, 13 strokes, DMF wash flow rate 40 mL/min. After the automated synthesis was completed (ca. 80 min), the peptidyl resin (0.24 g) was cleaved following the general Cleavage Protocol furnishing crude peptide as a colorless powder (91 mg). Purification of the total crude peptide in multiple portions following the method described in Semi-preparative Reverse-Phase High Performance Liquid Chromatography (RP-HPLC) furnished the desired peptide as a mixture of diastereomers (32 mg, 8.5 µmol; M = 3749.76 g/mol [Photo-OXB + {5 × 114.02 g/mol TFA}], >95% purity for both diastereomers) as a colorless powder in 26% overall yield.

### Cell culture, in vitro imaging, uncaging and quantification

HEK293T cells (ATCC #CRL-3216) were cultured in Dulbecco's Modified Eagle Medium (DMEM) containing 4.5 g/L D-glucose, L-Glutamine and Pyruvate (ThermoFisher Scientific) supplemented with 10% Fetal Bovine Serum (ThermoFisher Scientific) and 1x Anti-Anti (ThermoFisher Scientific) at 37 °C, 5% (v/v) CO₂. Cells were transfected at 40-50% confluency in 35 mm glass-bottomed dishes using Effectene^{®} Transfection Reagent (QIAgen) according to manufacturer instructions, and used for experiments -36-48 h after transfection. Cells were imaged at room temperature in 100 µl Hank's Balanced Salt Solution (HBSS) containing CaCl₂ and MgCl₂ (ThermoFisher Scientific) per 35 mm glass-bottom dish. Imaging was performed on an inverted Zeiss LSM 800 confocal microscope using a 40X Plan-Apochromat oil-based objective and a 488 nm laser for OxLight1, a 560 nm laser for jRGECO1a or a 647 nm laser for Alexa-647 conjugated anti-FLAG antibody, while a 405 nm laser was used for optical uncaging. Uncaging was performed using a 40x Plan-Apochromat oil-based objective (numerical aperture, N/A = 1,4; 69% transmittance at 405 nm) over a surface area of 1696 µm² with a scanning rate of 7 Hz and a pixel dwell time of 4.12 µsec. The average intensity of laser light used for uncaging was measured using a S120C Photodiode Power Sensor (Thorlabs) and was set to 0.38 mW. Ligand applications at the specified concentrations were performed by manual application during time-lapse imaging. For quantification of the dynamic range ΔF/F₀, regions of interest (ROIs) that enclose isolated cell membranes (for OxLight1) or cytoplasm (for jRGECO1a) were selected manually using the threshold function of Fiji. Sensor responses (ΔF/F₀) were calculated as follows: (F(t) - Fo)/Fo with F₍ₜ₎ being the ROI mean grey value at each time point (t), and F₀ being the mean grey value of the ten time points immediately prior to ligand addition or uncaging. For generating the signal-to-noise-ratio (SNR) heatmaps, ΔF/F₀ images were divided pixel-wise by the square root of the baseline fluorescence image, which was uniformly incremented by an arbitrary single digit value to avoid discontinuity during image division.

### Animals

Wild-type C57BL/6 mice aged between 6-24 weeks were used in this study. Mice were housed in a temperature- and humidity-controlled environment with 12 h light/12 h dark cycle (lights on at 7 AM). Weights and genders were distributed homogeneously among the groups.

### Slice preparation, electrophysiology, and uncaging

Coronal 250 µm slices containing NAc were prepared in ice-cooled artificial cerebrospinal fluid (sCSF) containing (in mM): NaCl 120, KCI 2.5, MgCl₂ 1.0, CaCl₂ 2.5, Na₂HPO₄ 1.25, NaHCO₃ 26.0, glucose 14.6 and HEPES 5.0, bubbled with 95% O₂ and 5% CO₂. Slices were kept at 34 °C for 10-20 minutes and then kept at room temperature until recordings. In the recording chamber, slices were superfused with aCSF at 30-32 °C. Visualized whole-cell patch-clamp recording techniques were used to measure the response to the uncaging of orexin. Patch pipettes (3-7 MΩ) were pulled from borosilicate glass pipettes (GC150T-10, Harvard Apparatus). The internal solution contained (in mM): potassium gluconate 130, MgCl₂ 4, MgATP 3.4, Na₃GTP 0.1, creatine phosphate 10, HEPES 5 and EGTA 1.1. The membrane potential was measured at 0 pA current injection using EPC 10 USB Patch Clamp Amplifiers (HEKA). The access resistance was monitored by a hyperpolarizing step of -10 mV. The data were excluded if the access resistance was above 20 MΩ. To evaluate the effect of uncaging of photo-OXB, UV LED light was applied with or without caged orexin (300 nM) to the brain slices on an upright Axio Examiner A1 microscope (Zeiss) using a Plan-Apochromat 63x/1.0 M27 objective (light source: Colibri 7, Zeiss; 385 nm wavelength with 30 nm bandwidth) at 2 mW/mm² intensity for 2 seconds with or without photo-OXB (300 nM). After the application of the UV, the slices were perfused with at least 5 mL of the aCSF to wash out uncaged photo-OXB before starting the recording of another neuron in the same brain slice.

### Electrophysiological identification of D1 and D2-MSNs

To measure input resistance and firing rate, negative (-200 to 0 pA, every 50 pA, 500ms) and positive currents (0 to 300 pA, every 50 pA, 500ms) were applied to the recorded neurons, respectively. It has been demonstrated that in the nucleus accumbens and in the dorsal striatum, the D2 receptor expressing medium spiny neurons (D2-MSNs) have higher input resistance and higher firing rate than D1 receptor expressing medium spiny neurons (D1-MSNs). Based on these findings, the higher input resistance (≈135 MΩ) and higher firing rate (≈30Hz at 200 pA injections) neurons were considered to be D2-MSNs and the lower input resistance (≈75 MΩ) and lower firing rate (≈5Hz at 200 pA injections) neurons were considered to be D1-MSNs.

### Cited prior art documents:

*R1:* Isidro-Llobet, A., Alvarez, M., & Albericio, F., Chem. Rev., 2009, 109(6), 2455-2504.

## Claims

1. A photocaged peptide P-PC, with P being a peptide and PC a photocleavable segment, which is coupled to the peptide at its C-terminus and wherein the photocleavable segment PC is a compound of formula (I) 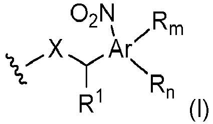 and wherein
X is NH or O,
Ar is an aromatic system selected from benzene, naphthalene, benzophenone, indole, benzothiophene, benzofuran, benzimidazole, quinoline, quinazoline, furan, thiophene, pyrrazole or pyrrole,
R¹ is a C₁-C₆-alkyl, -O-C₁-C₆-alkyl or a separated linker SL, particularly C₁-C₆-alkyl or the separated linker SL,
n is 0, 1, 2 or 3, particularly 1 or 2,
Rₘ is the separated linker SL and m is 0 or 1,
wherein at least one of R¹ or Rₘ is the separated linker SL,
Rₙ is selected from
- -O-C₁₋₆, -N-C₁₋₃, -S-C₁₋₃,
- the group of halogens,
- C₅-C₆-aryl, C₅-C₆-heteroaryl,
- C₂-C₉-conjugated alkenes, C₂-C₉-conjugated alkynes,
SL is -Y-SP-E, wherein Y is O, S, NR³ or CR³₂ and wherein R³ is H and/or a C₁-C₂ alkyl and wherein SP is a spacer comprising up to eight atoms, particularly carbon atoms, and wherein E is selected from -CONH₂, -COOH, -OH, -COSH, -COS-C₁-C₃-alkyl, -COS-C₅-C₆-aryl, -CONH-NH₂, -COO-C₁-C₄-alkyl, -COO-C₅-C₆-aryl, -CONH-C₁-C₂-alkyl, -CON-(C₁-C₂-alkyl)₂.

2. The photocaged peptide P-PC according to claim 1, wherein the photocleavable segment PC is a compound of formula (II) 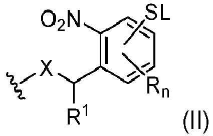 and wherein
X is NH or O,
R¹ is C₁-C₆-alkyl,
n is 0, 1, 2 or 3, particularly 1 or 2, particularly 1,
Rₙ is selected from
- -O-C₁₋₆, -N-C₁₋₃, -S-C₁₋₃,
- the group of halogens,
- C₅-C₆-aryl, C₅-C₆-heteroaryl,
- C₂-C₉-conjugated alkenes, C₂-C₉-conjugated alkynes,
SL is -Y-SP-E, wherein Y is O, S, NR³ or CR³₂ and wherein R³ is H and/or a C₁-C₂ alkyl and wherein SP is a spacer comprising up to eight atoms, particularly carbon atoms, and wherein, and wherein E is selected from -CONH₂, -COOH, -OH, -COSH, -COS-C₁-C₃-alkyl, -COS-C₅-C₆-aryl, -CONH-NH₂, -COO-C₁-C₄-alkyl, -COO-C₅-C₆-aryl, -CONH-C₁-C₂-alkyl, -CON-(C₁-C₂-alkyl)_{2.}

3. The photocaged peptide P-PC according to any of the preceding claims, wherein R¹ is selected from C₁*-*C₆ alkyl, particularly from C₁-C₃ alkyl, more particularly from methyl or ethyl, even more particularly R¹ is methyl.

4. The photocaged peptide P-PC according to any of the preceding claims, wherein Y is O, NR³ or CR³₂, particularly O or CR³₂, more particularly O.

5. The photocaged peptide P-PC according to any of the preceding claims, wherein E is selected from -CONH₂, -COOH, -OH, -COO-C₁-C₄-alkyl, or -COO-C₅-C₆-aryl, -COS-C₁-C₄-alkyl, or -COS-C₅-C₆-aryl, particularly from -CONH₂, -COOH, -OH or -COO-C₁-C₄-alkyl, more particularly E is -CONH₂ or -COOH.

6. The photocaged peptide P-PC according to any of the preceding claims, wherein Rₙ is selected from
- -O-C₁₋₆, -N-C₁₋₃,
- the group of halogens,
- C₂-C₉-conjugated alkenes, C₂-C₉-conjugated alkynes, particularly C₂-C₉-conjugated alkenes,
particularly Rₙ is selected from
- -O-C₁₋₆, -N-C₁₋₃,
- C₂-C₉-conjugated alkenes,
more particularly Rₙ is selected from
- -O-C₁₋₆, -N-C₁₋₃,

7. The photocaged peptide P-PC according to any of the preceding claims, wherein Rₙ is -O-C₁₋₆ or -N-C₁₋₃, particularly -O-C₁₋₃, or -N-C₁₋₂, more particularly Rₙ is -O-C₁₋₃.

8. The photocaged peptide P-PC according to any of the preceding claims, wherein Rₙ is -O-C₁₋₂, particularly Rₙ is OMe.

9. The photocaged peptide P-PC according to any of the preceding claims, wherein the photocleavable segment is a compound of formula (III), (IV), (V) or (VI), particularly of (III) or (V), more particularly of (III).

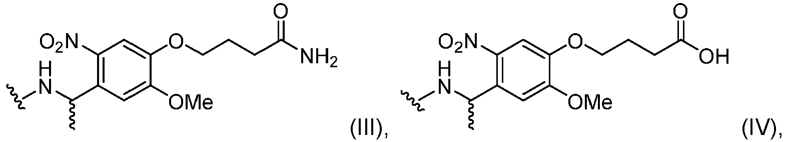

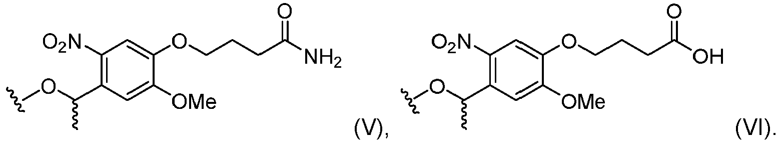

10. The photocaged peptide P-PC according to any of the preceding claims, wherein the peptide P comprises a C-terminal amide or C-terminal carboxylic acid.

11. The photocaged peptide P-PC according to any of the preceding claims, wherein the peptide P is a receptor binding peptide, particularly a GPCR binding peptide.

12. The photocaged peptide P-PC according to any of the preceding claims, wherein P is selected from cholecycystokinin, gastrin, prolactin-releasing peptide, pyroglutamylated RF amide peptide, neuropeptide FF, kisspeptin, vasopressin, oxytocin, bombesin, gastrin-releasing peptide, neuromedin U, substance P, neurokinin, thyrotropin-releasing hormone, orexin A, apelin, endotelin, compliment ligand C3a, compliment ligand C5a, gonadoptrin releasing hormone, PDZ ligands or orexin B, particularly from neuropeptide Y, vasopressin, oxytocin, orexin A or orexin B, more particularly the P is orexin B.

13. A method for synthesising the photocaged peptide P-PC particularly according to claims 1 to 13, wherein the method is **characterised in that**
• an Fmoc protected amino acid building block (AA) is coupled to a double linker, wherein the double linker comprises a photocleavable segment precursor (PCP) of formula (VII), **characterised in that** it comprises a terminal moiety allowing the connection to a resin linker (RL) 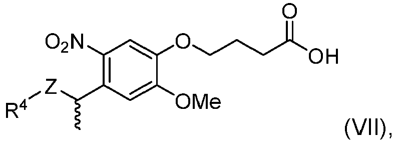 wherein Z is O or NH,
R⁴ is Fmoc or H,
• and the resin linker (RL), wherein in particular the resin linker is a a Rink amide linker, HMPB, Fmoc-PAL-OH or Fmoc-MeDBz-OH particularly Rink amide or HMPB, yielding a linked amino acid building block AA-PCP-RL,the linked amino acid building block is deprotected and coupled in continuous coupling and deprotection steps via automated solid-phase peptide synthesis, yielding a photocaged peptide precursor P-PCP-RL,
• the photocaged peptide precursor is decoupled from the resin linker, yielding the photocaged peptide P-PC,
wherein all definitions are identical to the definitions of claims 1 to 13.

14. The method for synthesising the photocaged peptide according to claim 14, wherein Z is NH and/or R⁴ is Fmoc.

15. The method for synthesising the photocaged peptide according to claims 14 and 17, wherein the double linker is coupled to the C-terminus of the amino acid building block.
